# EUROPEAN PATENT APPLICATION

(11) **EP 1 002 805 A1**
(43) Date of publication of application: **24.05.2000**
(21) Application number: 99919609.0
(22) Date of filing: 17.05.1999
(51) Int. Cl.: C08B 37/00

(54) **FIXATION INHIBITORS FOR HELICOBACTER PYLORI**

(30) Priority: 09.06.1998 JP 16053198
(71) Applicant: Ghen Corporation, Gifu-shi Gifu-ken 501-1132 (JP); Nisshin Flour Milling Co., Ltd., Chiyoda-ku, Tokyo 101-8441 (JP)
(72) Inventor: KODAMA, Yoshikatsu, Gifu 501-1171 (JP); KIMURA, Nobutake, Nisshin Flour Milling Co. Ltd., Ohi-machi, Iruma-gun, Saitama 356 (JP)
(74) Representative: Warcoin, Jacques
(86) International application number: JP9902540
(87) International publication number: WO9964469

(57) **Abstract**

An inhibitor of Helicobacter pylori colonization includes as an active ingredient a polysaccharide having sulfate groups. This substance can eliminate specifically Helicobacter pylori from the stomach and so can provide safe and effective pharmaceutical compositions for use in the prevention or treatment of gastritis, gastric ulcers, etc., and provide a food for prevention or treatment of these diseases. This substance may be used along with an inhibitor of gastric acid secretion to eliminate Hp more effectively.

## Description

### TECHNICAL FIELD

The present invention relates to inhibitors of the colonization of Helicobacter pylori (hereinafter referred to as Hp), pharmaceutical compositions orally administrable for the prevention or treatment of gastritis, gastric ulcers and duodenal ulcers caused by infection of Helicobacter pylori, and foods useful for the prevention or treatment of gastritis, gastric ulcers and duodenal ulcers.

### BACKGROUND ART

At present, energetic studies on infectious diseases caused by Helicobacter pylori are being performed in the clinical field of the digestive system. This is because this bacterium plays an important role in the cause of gastric disorders such as gastritis and gastric ulcers, and about half of the population of the world are infected with this bacterium. Therefore, the International Agency for Research on Cancer (IARC) of WHO (the World Health Organization) recognizes Helicobacter pylori as a definite carcinogen based on epidemiological studies.

Helicobacter pylori is a gram-negative spiral rod-shaped bacterium having some flagella at one end and inhabiting the human gastric mucosa. Marshall, B.J. and Warren, J.R. in Australia reported in 1983 that this bacterium was frequently detected in stomach biopsy specimens from patients with gastric ulcers. Since then, many reports have been published based on epidemiological studies, indicating that this bacterium causes gastritis, gastric ulcers and duodenal ulcers and is associated with diseases such as gastric cancer.

Virulence factors of Hp infectious diseases have been considered to include urease produced by Hp, flagella for moving freely in the mucosal mucin layer, Cag A outer membrane protein involved in the production of interleukin 8 as an inflammatory cytokine, Vac A vacuolating cytotoxin concerned in vacuolation, erosion, necrosis and ulceration of gastric mucosal epithelial cells. One of these virulence factors, urease produced by Hp, has been considered to provide an environment useful for growth of Hp in the stomach by producing ammonia, which neutralizes a strongly acidic environment in the stomach and damages gastric mucosa, thereby causing gastritis or gastric ulcers and finally causing gastric cancer.

It is believed that eradication of Helicobacter pylori from the stomach is essential for the treatment of peptic ulcers. To eradicate Helicobacter pylori, a combination of antibiotics and a suppressor of gastric acid secretion is commonly used. Once Hp colonizes gastric mucosa, it is difficult to eradicate Hp from the stomach only with antibiotics. Therefore, a combination of some antibiotics and a proton pump inhibitor which strongly suppresses the secretion of gastric acid is utilized often in a greater dose than usual for eradication of Hp. However, the administration of antibiotics for a long period of time has the serious problems of increasing antibiotic-resistant strains as well as causing side effects.

At present, an immunological therapy approach using an oral vaccine has been proposed in order to solve problems such as side effects and increase of antibiotic-resistant strains by treatment with antibiotics for the eradication of the bacteria. However, model animals for Hp infection and complicated conditions for experiments are required for this purpose. These requirements have obstructed studies aimed at developing new methods for prevention and treatment. Furthermore, the oral vaccine method has unsolved problems in respect to safety of adjuvands, which are essential for oral vaccines, in its practical application to humans. Also, the vaccine is predominantly used for prevention, and therefore it has no effect on patients who have already been infected with Hp.

As a new attempt in the form of an immunological therapy instead of a vaccine method, the use of specific antibodies is proposed, which antibodies are obtained from the eggs of hens immunized against Hp whole cells as an antigen. However, it cannot be expected to eliminate Hp completely from the stomach by using antibodies against whole cells of Hp. Also, there is no evidence as to whether these antibodies can actually eliminate Hp from the stomach.

On the other hand, it is disclosed that cells of certain bifid bacteria or lactic acid bacteria, and polysaccharides extracted from these cells are useful in prevention or treatment of gastric ulcers (Japanese Patent Application Kokai No. 4-5236), and that polysaccharide of rhamnose, rramnan, derived form certain seaweed and olygosaccharide of rhamnose are useful as an antiulcer agent (Japanese Patent Application Kokai No. 6-247861). Japanese Patent Application Kokai No. 7-138166 describes the use of fucoidan, which is a polysaccharide derived from Nemacystus. This publication states that fucoidan inhibits the colonization of Hp in gastric mucosa and has antiulcer activity. However, ulcers induced with acetic acid, which are basically different from Hp-induced ulcers with respect to pathological development, are used in order to show the effects of treatment of ulcers in that publication. Therefore, in that publication, there is no evidence for suppressing the formation of gastritis or ulcers caused by Hp infection. Furthermore, that publication states that fucose (monosaccharide) is considered to be a colonization factor (adhesin), and an in vitro experiment based on this assumption was performed using biotinylated fucose as an adhesion marker to see an inhibitory effect of fucoidan on Hp colonization. However, fucose is not considered to be an adhesin at present, so that experiment does not show an inhibitory effect of fucoidan on Hp colonization.

Also, the above-mentioned antiulcer agents or inhibitors are not based on the elucidation of the mechanism of Hp colonization. The mechanism of Hp colonization has not been elucidated. More particularly an adhesin of Hp and a receptor of the adhesin on the gastric mucosa are unknown prior to the present invention. Accordingly, effective inhibitors of Hp colonization or effective antiulcer agents have not been developed based on a finding with respect to the mechanism of Hp colonization. For example, the above-mentioned Japanese Patent Application Kokai No. 4-5236 does not teach which kind of polysaccharide exhibits antiulcer activity, and furthermore that publication relates to the prevention or treatment of acetic acid-induced ulcers and alcoholic stress-induced ulcers. Also, the polysaccharides used in Japanese Patent Application Kokai No. 6-247861 are desulfated polysaccharides, and this is contrary to the discovery of the present invention that sulfate groups take part in the inhibition of Hp colonization.

The above-mentioned Japanese Patent Application Kokai No. 7-138166 describes the use of fucoidan comprising a major of fucose (monosaccharide) and a minor of uronic acid, a part of constitutive monosaccharides being sulfated, and states that the fucoidan exhibits an inhibitory effect on Hp colonization. However, that publication does not teach or suggest that the sulfate group in polysaccharide is effective for inhibition of Hp colonization, so it does not provide a medicine based on the mechanism of inhibition of Hp colonization. Moreover, that publication has no evidence showing that fucoidan has an inhibitory effect on Hp colonization as mentioned before.

As explained above, the long-term use of antibiotics for elimination of Hp results in an increase in antibiotic-resistant bacteria as well as side effects, and a vaccine and egg antibodies against Hp whole cells do not exhibit enough efficacy. The mechanisms on which the antiulcer activity of some polysaccharides and the inhibitory activity of fucoidan on Hp colonization are based have not been made clear.

### DISCLOSURE OF THE INVENTION

It is an object of the present invention to provide an inhibitor pf Hp colonization by elucidating the mechanism of Hp colonization in the stomach which is associated with the occurrence of peptic ulcers, and to provide a pharmaceutical composition for use in preventing or treating gastritis, gastric ulcers, etc. caused by Hp infection, the composition being effective and safe without the disadvantages of side effects and increase of drug-resistant strains, and to provide a food substance for prevention and treatment of these diseases.

The present inventors have found the mechanism of colonization of Hp to gastric mucosa, which mechanism has not previously been elucidated fully, and completed the present invention based on this discovery.

Due to the specificity of growth conditions, the ecology of Hp in the stomach has not been elucidated. Especially, the reason for strong growth ability in the stomach could not be explained, although various studies have been conducted on the colonization of Hp to gastric mucosa, which is the key of growth in the stomach , which has strong acidity. The adhesion of Hp to gastric mucosa plays an important role in the growth of Hp in the stomach, and therefore elucidation of its adhesins is of great significance in developing a method for preventing or treating gastritis and peptic ulcers caused by Hp.

Urease produced by Hp, one of the virulence factors which have been proposed, has been considered to act as an enzyme which converts urea in the stomach to ammonia and thereby causes gastritis, gastric ulcers or gastric cancer, as mentioned before. Also, various experiments were reported showing that urease does not take part in the colonization of Hp in gastric mucosa, that is to say that urease does not function as an adhesin. The present inventors have made the discovery, not expected from the results of prior art studies on the adhesion of Hp, that urease itself participates in the adhesion of Hp in the gastric mucosa. That is to say, the inventors have found that urease produced by Hp functions as a main adhesin in addition to functioning as an enzyme capable of converting urea to ammonia, and that the binding of urease itself to gastric mucosal mucin enables the growth of Hp.

Also, the inventors obtained information suggesting that the receptor of urease in gastric mucosa mucin has sulfate groups which play a role in the receptor. Thus, by making clear the relation between the adhesin and the receptor thereof, a new method for eradication of Hp from the stomach can be provided without the occurrence of antibiotic-resistant strains.

In one aspect, the present invention provides an inhibitor of Helicobacter pylori colonization, comprising a polysaccharide having sulfate groups as an active ingredient, the polysaccharide being other than polysaccharides comprising at least 50 mole % of fucose and having sulfate groups. The present invention also provides an inhibitor composition of Helicobacter pylori colonization, comprising a polysaccharide having sulfate groups and an inhibitor of gastric acid secretion as active ingredients.

In another aspect, the present invention provides a pharmaceutical composition containing as an active ingredient a polysaccharide having sulfate groups, the polysaccharide being other than polysaccharides comprising at least 50 mole % of fucose and having sulfate groups, for use in preventing or treating gastritis, gastric ulcers and duodenal ulcers caused by infection of Helicobacter pylori. The present invention also provides a pharmaceutical composition containing as active ingredients a polysaccharide having sulfate groups and an inhibitor of gastric acid secretion for use in preventing or treating gastritis, gastric ulcers and duodenal ulcers caused by infection of Helicobacter pylori.

The present invention also provides a food substance containing a polysaccharide having sulfate groups, the polysaccharide being other than polysaccharides comprising at least 50 mole% of fucose and having sulfate groups, for use in preventing or treating gastritis, gastric ulcers and duodenal ulcers.

The polysaccharide having sulfate groups used in the present invention is preferably dextran sulfate, κ-carrageenan, λ-carrageenan, heparin, or sulfated curdlan.

### BRIEF DESCRIPTION OF THE DRAWINGS

FIG. 1 is a graph showing adhesion patterns of urease to purified gastric mucin and MKN45 cells.
FIG.2 is a graph showing the adhesion of unease to MKN45 cells.
FIG.3 is a graph showing the inhibition rate of urease adhesion by various polysaccharides having sulfate groups.
FIG.4 is a graph showing the inhibition rate of urease adhesion by various polysaccharides having sulfate groups.

### BEST MODE FOR CARRYING OUT THE INVENTION

In carrying out the present invention, polysaccharides having sulfate groups can be used as an inhibitor of Hp colonization. These polysaccharides may be naturally occurring or chemically synthesized. The term "polysaccharides" as used herein means carbohydrates comprising more than a few monosaccharides, including oligosaccharides, and includes homopolysaccharides, heteropolysaccarides, and polysaccharides which constitute complex carbohydrates. The sulfate group may be an O-sulfate group or N-sulfate group (sulfoamino group) and may be located in any position of the monosaccharide.

The sulfate group content in the sulfate group-containing polysaccharide used in the present invention may be usually at least 5% as sulfur, and preferably 9-20%. Most preferably, polysaccharides having a sulfur content of about 10-18% are used, since the inhibition ability of Hp colonization to gastric mucosa and the sulfur content are correlated.

Examples of sulfate group-containing polysaccharides used in the present invention include dextran sulfate, chondroitin sulfate, heparin, heparinoid, mannan sulfate, charonin sulfuric acid, carrageenan (e.g. κ-carrageenan, λ-carrageenan), dermatan sulfate, funoran, porphyran, etc. as naturally occurring polysaccharides, and sulfated curdlan, sulfated lactose, sulfated oligosaccharides, sulfated lentinan, sulfated schizophyllan, sulfated yeast glucan etc. as synthetic polysaccharides. Dextran sulfate, κ-carrageenan, λ-carrageenan, heparin are preferred, and dextran sulfate and λ-carrageenan are more preferred as naturally occurring polysaccharides, and sulfated curdlan is preferred as a synthetic polysaccharide.

The sulfate group-containing polysaccharides used in the present invention may be commercially available products. For example, chondroitin sulfate and carrageenans are known as food additives (thickening agents) and dextran sulfate and heparin are known as materials for preparing anticoagulants for subcutaneous, intramuscular or intravenous injection. As sulfate group-containing polysaccharides, extracts from animal tissues or marine algae such as Rhodophyta (red algae) and Phacophyceae (brown algae) containing the sulfate group-containing polysaccharides, or purified products thereof may be used, or naturally-occurring polysaccharides having less content of sulfate groups or no sulfate group may be sulfated and used. Methods for extracting, purifying and sulfating may be any conventional methods.

The inhibitor of Hp colonization comprising sulfate group-containing polysaccharides as an active ingredient and the antiulcer agent containing the inhibitors may be administered by an oral route. Some sulfate group-containing polysaccharides have anticoagulation activity and therefore have side effects when administered by injection. However, these have no side effects and are safe when administered by an oral route. Some polysaccharides used as food additives are, of course, confirmed to be safe.

As is apparent from the in vitro experiments and animal experiments mentioned below, the sulfate group-containing polysaccharides used in the present invention are capable of inhibiting Hp colonization to gastric mucosa and exhibit remarkable effects of eliminating Hp from the stomach. Therefore, pharmaceutical compositions comprising sulfate group-containing polysaccharides as an active ingredient are useful in preventing or treating gastritis, gastric ulcers and duodenal ulcers caused by colonization of Hp in gastric mucosa, and foods containing sulfate group-containing polysaccharides can be used for prevention or treatment of gastritis, gastric ulcers and duodenal ulcers.

In the past, urease produced by Hp infection had been noticed as one of the virulence factors for pathologic development of gastritis or gastric ulcers by Hp infection, but urease had never been thought to be a factor associated with adhesion of Hp to the gastric mucosa. Under these circumstances, the present inventors previously found that an adhesin of Hp is urease localized on the surface layer of Hp cell, and proposed the use of anti-urease antibodies from eggs for prevention or treatment of gastritis, gastric ulcers, etc. (Japanese Patent Application Kokai No. 10-287585). The inhibitory ability of the anti-urease antibodies for Hp adhesion was studied by utilizing the fact that Hp adheres well to human gastric cancer-derived MKN-45 cells and secretes an inflammatory cytokine, interleukin 8. Furthermore, the inhibitory ability of the anti-urease antibodies for Hp colonization was also studied in hairless mice having a high sensitivity to Hp infection. As a result, these antibodies inhibited adhesion of Hp to MKN cells effectively, and showed significant inhibitory effects on Hp colonization in animal experiments. Unexpectedly, these anti-urease antibodies can hardly inhibit enzyme activity of urease. This suggested that urease participates in the adhesion of Hp to the gastric mucosa in addition to functioning as an enzyme, which was known in the past.

The present inventors have furthermore showed that biotinylated urease specifically adheres to heparin, gastric mucin or MKN-45 cells, and demonstrated that the adhesion of urease can be inhibited specifically by sulfate group-containing polysaccharides and that Hp colonization in the stomach can be inhibited dose-dependently and specifically by oral administration of sulfate group-containing polysaccharides in animal experiments (as shown in Experiments below). From these findings, it is apparent that urease of Hp not only damages the mucosa by forming ammonia in the stomach but functions as an adhesin. Also, it is supposed that the receptor molecule in gastric mucin, which receptor is to urease, an adhesin of Hp, contains a sulfate group or sulfate ester.

The present invention provides effective inhibitors of Hp colonization, pharmaceutical compositions for use in prevention or treatment of gastritis, gastric ulcers and duodenal ulcers, and foods for prevention or treatment of the same, based on the clarification of the relation between the adhesin of Hp and the receptor thereof, and the confirmation that sulfate group-containing polysaccharides have an inhibitory activity on Hp colonization.

When the sulfate group-containing polysaccharide is used as an inhibitor of Hp colonization, or as an active ingredient of pharmaceutical composition for prevention or treatment of gastritis, gastric ulcers, etc., the polysaccharide may be in any form suitable for oral administration. Usually, the sulfate group-containing polysaccharides as a major ingredient may be formulated together with a conventional carrier or excipient to form a pharmaceutical composition by any method. If necessary, other additives or agents may be added. For example, antacids (e.g., sodium hydrogencarbonate, magnesium carbonate, precipitated calcium carbonate, synthetic hydrotalsite), agents for protection of gastric mucosa (e.g., synthetic aluminium silicate, sucralfate, and sodium copper chlorophyllin) and digestive enzymes (e.g., biodiastase or lipase) may be added to the pharmaceutical composition. The dosage of the sulfate group-containing polysaccharide is not limited, but preferably is 1-5 mg/kg per day for an adult in the case of prevention and 50-500 mg/kg per day for an adult in the case of treatment.

When the sulfate group-containing polysaccharide is used in food for prevention or treatment of gastritis, gastric ulcers and duodenal ulcers, the polysaccharide may be contained in an amount of at least 0.1%, preferably at least 0.5%.

As inhibitors of gastric acid secretion, H₂ inhibitors such as cimetidine, ranitidine and famotidine and proton pump inhibitors such as omeprazol and lansoprazol may be used. The dosage of the inhibitor of gastric acid inhibitor is preferably 20-30 mg per day per an adult.

The following examples are given to further illustrate the present invention. It should be understood that the present invention is not limited to the specific details set forth in the examples.

### EXAMPLES

### Example 1

### Formula 1: in 1.5g of fine particles

| | |
|---|---|
| dextran sulfate | 100 mg |
| lactose | 950 mg |
| corn starch | 405 mg |
| PVP(K-30) | 45 mg |

These components were formulated into fine particles by a conventional wet granulation method.

### Formula 2: in three tablets

| | |
|---|---|
| γ-carrageenan | 300 mg |
| lactose(200m) | 250 mg |
| corn starch | 50 mg |
| crystallized cellulose | 180 mg |
| PVP(K-300) | 22 mg |
| magnesium stearate | 8 mg |

These components were formulated into tablets by a conventional method.

### Formula 3: in 1.5g of granules

| | |
|---|---|
| sulfated curdlan | 100 mg |
| lactose(200M) | 900 mg |
| corn starch | 450 mg |
| PVP(K-30) | 50 mg |

These components were formulated into granules by a conventional extrusion granulation method.

### Experiment 1 In vitro Experiment

Inhibitory effects on colonization of urease produced by Hp in gastric mucosa were examined in an in vitro experiment system using various sulfate group-containing polysaccharides.

### (Materials and Methods)

### Adhesion test of urease to gastric mucin

The present inventors had already found that an adhesin of Hp is urease produced by Hp. The urease binds well to mucin of gastric mucosa, as shown below.

Porcine gastric mucin to be used for a urease adhesion test was prepared as follows.

Healthy pigs about two months old were slaughtered, and their stomachs were recovered and washed on the insides thereof with PBS (pH 7.4) containing 0.1M phosphate + 0.15M NaCl + 5mM N-ethyl maleimide (NEM) + 1mM phenylmethylsulfonyl fluoride (PMSF) + 1mM EDTA. The stomachs were incised, and gastric mucosa was scraped and suspended in the above-mentioned buffer. This suspension of mucosa was homogenized by a Polytron homogenizer while being iced and was centrifuged at 15,000xg to recover supernatant. The supernatant was centrifuged again at 25,000xg to recover supernatant, which was dialyzed against distilled water and lyophilized to obtain crude gastric mucin. Then, this lyophilized crude gastric mucin was dissolved in PBS (pH 6.8) containing 6M guanidine hydrochloride and protease inhibitor (5mM NEM, 1mM PMSF, 1mM EDTA), and overlaid on a cesium chloride density gradient (1.5 g/ml) and centrifuged at 34,000xg for 48 hours. A sialic acid-containing fraction was detected by nitrocellulose membrane blotting and dyeing with periodic acid Schiff's reagent. Dyed fractions were pooled and overlaid on a cesium chloride density gradient and centrifuged. Dyeing-positive fractions were pooled and lyophilized. Then, the lyophilized product was subjected to gel filtration through Sepharose CL-4B column equilibrated with 0.1M phosphate buffer (0.1M NaCl, pH 6.8) to carry out fractionation. Fractions which were PAS dyeing-positive and had proteins at a high concentration were pooled and dialyzed against PBS (pH 6.8) to obtain purified porcine gastric mucin, which was stored at -80°C until use. The obtained gastric mucin was confirmed to be glycoprotein of 66kD by SDS-PAGE.

A microplate for a urease adhesion test was prepared as follows.

To each well of a 96 well microplate, a 100 µl portion of 1.25% glutaraldehyde solution was added, and sensitization was conducted for 5 minutes. After washing each well three times with distilled water, a 50 µl portion of purified porcine gastric mucin (1.27 mg/ml) was added to each well and was subjected to immobilization by standing overnight at 4°C. When the microplate was used, blocking was conducted by adding 3% BSA to each well to react at 37°C for 60 minutes, and then the plate was washed three times with PBS supplemented with 0.05% Tween 20.

A urease adhesion test was carried out using the microplate prepared above as follows.

Purified biotinylated urease was diluted so as to give a final concentration of 7.0 µg/ml with adhesion media having different pH ranges (20mM phosphate buffer containing 0.01% Tween 20 and 0.15M NaCl, pH adjusted to be 2.0, 3.0, 4.0, 4.5, 5.0, 5.5, 6.0 or 6.5). Each urease sample thus prepared was added to 2 wells of the mucin-immobilized microplate mentioned above to conduct sensitization at 37°C for 60 minutes. Then, immediately after each well was washed three times with the adhesion medium, 10% neutral formalin (pH 7.4) was added to each well, and the plate was allowed to stand at 37°C for 30 minutes to perform fixation. In order to determine the amount of urease adhered to the well, streptoavidin HRP was added to each well to react at 37°C for 60 minutes. Then, ortho-phenylenediamine 2HCl as a substrate and H₂O₂ were added to react. 3N H₂SO₄ was used for termination of the reaction. Known amounts of urease diluted serially 2-fold were placed in a running plate and a calibration curve thereof was used to determine the amount of urease in a sample.

### Urease adhesion test to human gastric cancer-derived MKN45 cells

It is disclosed that Hp adheres well to human gastric cancer-derived MKN45 cells and interleukin-8 is secreted (Kabir,A.M.A. et al., 1997, Gut 40:49-55)(Cell were obtained from the Medical College of Tohkai University). Using these cells adhesion ability of biotynylated urease was examined in a similar way as in the above-mentioned purified gastric mucin.

Monolayer cultured MKN cells were obtained by inoculating cell suspension to each well of 96 well microplate so as give a 2x10⁴ cells per well and culturing for about 7 days in a 5 % CO₂ incubator at 37°C to obtain monolayer cultured cells.

### Inhibition test of urease adhesion

Inhibition tests of urease adhesion by various polysaccharides were conducted using human gastric cancer-derived MKN45 cells. First, samples (sulfate group-containing polysaccharides) having various concentrations were each mixed with biotinylated urease, and each mixture was shaken at 37°C for 60 minutes to carry out sensitization. Then, this mixture was transferred to monolayer cultured cells of each well in a 96 well-microplate, and the plate was shaken at 37°C for 60 minutes to carry out sensitization. Then, each well in the microplate was washed three times with adhesion medium (pH 3.0) and was fixed by heating at 65°C for 10 minutes. The fixed wells were washed three times with PBS-Tween 20 (0.5%) (pH 6.8), and strepto-avidin HRP was added to each well, and biotinylated urease adhered to the cells was detected by ELISA described in the above "Adhesion test of urease to gastric mucin".

### Results

### Urease adhesion pattern to purified gastric mucin and MKN45 cells

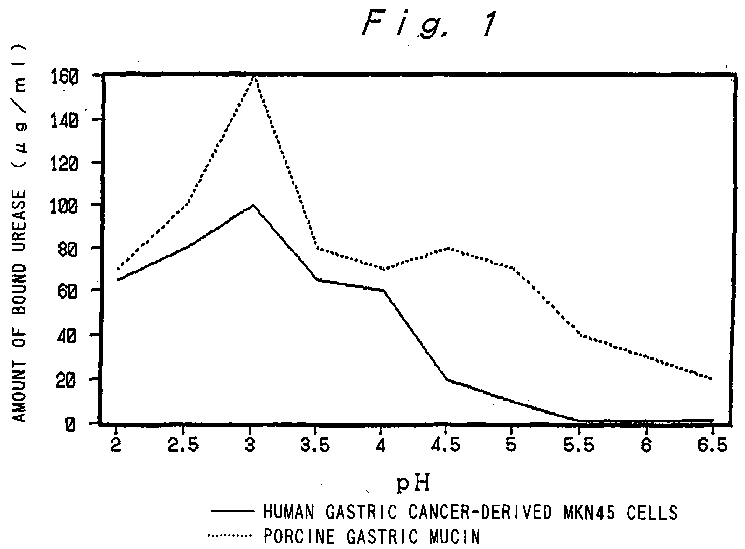

As shown in Fig. 1, the adhesion pattern of urease to purified gastric mucin is similar to the pattern of adhesion to MKN45 cells. That is to say, urease adhesion to MKN45 cells depends on pH, as is the case with the gastric mucin, suggesting that MKN45 cells express on the cell surface the same receptors as those recognized in gastric mucin (The urease adhesion pattern to gastric mucin is somewhat different from that to MKN45 cells in that shoulderpeak is observed at about pH 4.5. However, this is because enough urease receptors may not be expressed on the surface of MKN45 cells, or receptor molecules may be somewhat different in their constitution. Since urease adhesion reaction at about pH 3.0 is considered to reflect the colonization character of Hp in gastric mucosa, a substance which is able to inhibit the adhesion of urease in this pH range may inhibit the colonization of Hp in the stomach, and therefore may be useful for preventing or treating gastritis and gastric ulcers.

### Urease adhesion to MKN45 cells

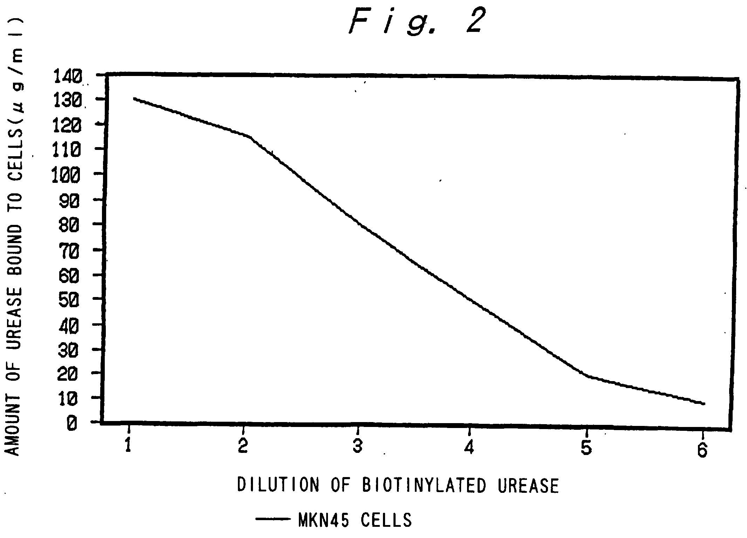

As is apparent from Fig.2, the urease adhesion reaction to MKN45 cells exhibits an S-shaped curve dose-dependently.

### Inhibition of urease adhesion with sulfate group-containing polysaccharides

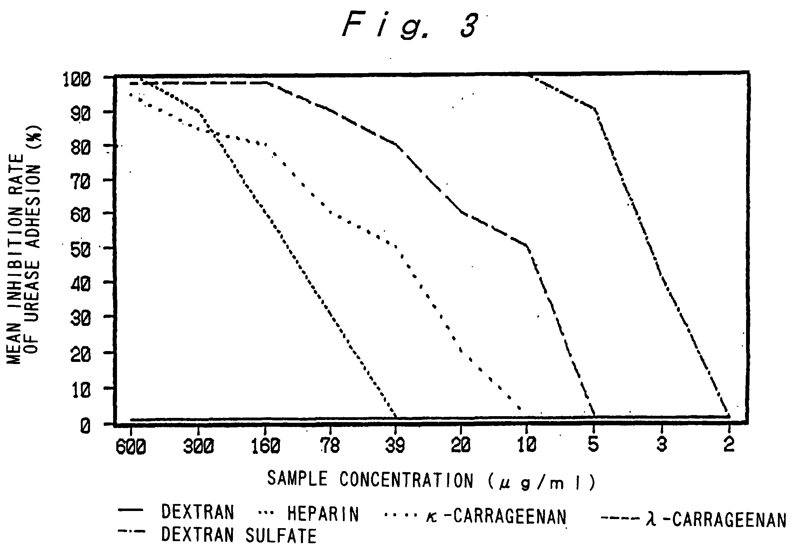

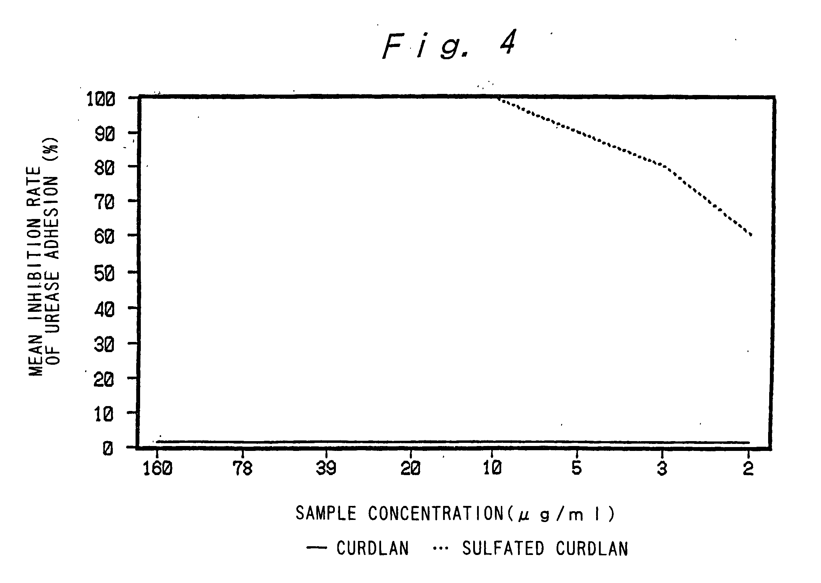

As shown in Fig. 3 and 4, urease adhesion reaction to MKN45 cells was specifically inhibited with sulfate group-containing polysaccharides. Fig. 3 shows the mean inhibition rate of urease adhesion in the case of dextran sulfate, heparin, λ-carrageenan, and κ-carrageenan as sulfate group-containing polysaccharide, indicating that these sulfate group-containing polysaccharides inhibit urease adhesion specifically and dose-dependently. On the other hand, sulfate group-free dextran as a control substance did not inhibit adhesion. Also, Fig. 4 shows the mean inhibition rate of urease adhesion in the case of curdlan as a control substance and sulfated curdlan as a sulfate group-containing polysaccharide, indicating that sulfated curdlan inhibits the adhesion specifically and dose-dependently.

From the above results, it is apparent that urease produced by Hp adheres to gastric mucin and MKN45 cells specifically and dose-dependently and that the urease adhesion is inhibited by sulfate group-containing polysaccharides specifically and dose-dependently. Oral administration of sulfate group-containing substance will be able to eradicate Hp by specifically binding to urease of Hp in the stomach, since urease is localized on the surface of Hp cells.

### Experiment 2 In vivo experiment

This experiment was performed to further confirm the results of Experiment 1.

The experimental animal was a hairless mouse (NS:Hr/ICR, Research Institute for Human and Animal Propagation, Accession No. IRA-NHI-9701)(ATCC #72024) having a high sensitivity to Hp infection. The experiment using animals was conducted based on the schedule indicated in the following Table 1. Each sample was dissolved in drink and freely administered from 2 days before challenge until sacrifice. The amount of the drink was 4-8 ml per day per mouse. Each mouse was challenged with 1x10⁹ CFU of NSP 335 (Cag A⁺ and Vac A⁺) isolated from human clinical material. After the completion of administration of the samples, the mice in each group were slaughtered. The stomachs of the mice were recovered, and after removal of the contents, the stomachs were washed eight times with PBS (pH 7.2) by a vortex mixer and homogenized by a homogenizer to form an emulsion, which was used for detection of Hp. The detection of Hp was carried out by placing the emulsion on a medium for detecting Hp (Poremedia Hp isolation medium, Eiken Kagaku), incubating at 37°C for 5 days by the gas pack method, and counting colonies.

The samples used were dextran sulfate, dextran, carrageenan, curdlan and curdlan sulfate.

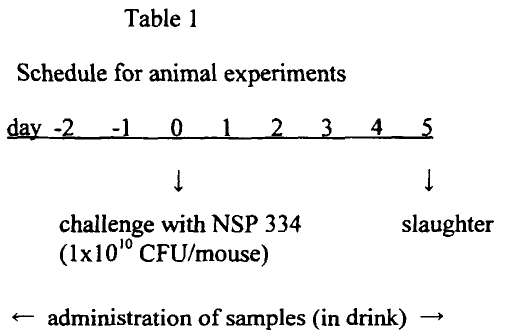

Tables 2-1 and 2-2 show the results of in vivo experiments. As is apparent from these results, sulfate-group containing polysaccharide (κ-carrageenan, λ-carrageenan, dextran sulfate, sulfated curdlan) inhibited the colonization of Hp in the stomach and exhibited remarkable effects of eliminating Hp from the stomach. Also, there seems to be correlation between the content of the sulfate group of the polysaccharide and the inhibitory ability of colonization (Table 2-2). On the other hand, the sulfate group-free polysaccharide could not inhibit the colonization of Hp as well as the non-administered control group.

**Table 2-1**

| sample | uninfected mice | % elimination rate | significance level |
|---|---|---|---|
| κ-carrageenan(250µg/ml) | 10/15 | 67 | P<0.001 |
| λ-carrageenan(250µg/ml) | 13/15 | 87 | P<0.001 |
| dextran sulfate(25µg/ml) | 19/20 | 95 | P<0.001 |
| dextran(25µg/ml) | 0/20 | 0 | NS |
| control | 0/20 | 0 | |

**Table 2-2**

| sample | amount (in drink) | uninfected mice | % elimination rate | significance level |
|---|---|---|---|---|
| sulfated curdlan | 0.1 µg/ml | 15/20 | 75 | P<0.001 |
| | 1.0µg/ml | 17/20 | 85 | P<0.001 |
| | 10.0µg/ml | 19/20 | 95 | P<0.001 |
| | 100.0µg/ml | 20/20 | 100 | P<0.001 |
| curdlan | 10.0µg/ml | 0/20 | 0 | NS |
| control | | 0/20 | 0 | |

### Experiment 3 In vivo experiment (Combination of sulfate group-containing polysaccharide and inhibitor of gastric acid secretion)

This experiment was conducted in animals to demonstrate the synergistic effects obtained by combination of a sulfate group-containing polysaccharide and an inhibitor of gastric acid secretion (H₂ inhibitor or proton pump inhibitor). In this experiment, as a sulfate group-containing polysaccharide, dextran sulfate and sulfated curdlan were used, these substance having exhibited high rate of elimination in Experiment 2.

The same procedure as in Experiment 2 was used except that the schedule of the experiments was as shown in the following Table 3 and the administration of the samples was from one week after challenge until slaughter.

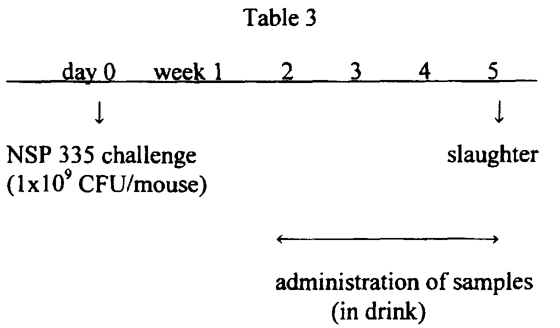

Table 4 shows the effects of eliminating Hp from the stomach when dextran sulfate or sulfated curdlan as a sulfate group-containing polysaccharide and cimetidine (H₂ inhibitor) or omeprazol (proton pump inhibitor) as an inhibitor of gastric acid secretion were used. Even though the administration was performed from one week after the challenge (when the colonization of Hp became stable), this combination exhibited a high elimination rate, which means that the combination is more effective than sulfate group-containing polysaccharide alone.

**Table 4**

| Administered Group | uninfected mice | % elimination rate |
|---|---|---|
| dextran sulfate (2.5µg/ml) +cimetidine (200µg/ml) | 8/10 | 80 |
| dextran sulfate (2.5µg/ml) +omeprazole (15µg/ml) | 9/10 | 90 |
| sulfated curdlan(0.1µg/ml) +cimetidine (200µg/ml) | 9/10 | 90 |
| sulfated curdlan(0.1µg/ml) +omeprazole (15µg/ml) | 10/10 | 100 |
| Control Group | 0/10 | 0 |

From the above results of animal experiments, it is apparent that sulfate group-containing polysaccharides can inhibit effectively the colonization of Hp in the stomach, and the combination of a sulfate group-containing polysaccharide and an inhibitor of gastric acid secretion such as a H₂ inhibitor or a proton pump inhibitor shows more effective effects of elimination. These substances can provide a useful method for eradication of Hp because unlike antibiotics because they have no problem of drug resistance.

### INDUSTRIAL APPLICABILITY

As mentioned above, the present invention provides effective inhibitor of colonization of Helicobacter pylori in the gastric mucosa. This inhibitor can be used in the form of safe and effective pharmaceutical compositions for use in prevention or treatment of gastritis, gastric ulcers, etc. caused by Hp infection, and also in the form of a food useful for prevention or treatment of these diseases. The present invention relates to the use of sulfate group-containing polysaccharides for inhibition of Hp colonization based on the discovery of a factor involved in the adhesion of Hp to gastric mucosa and a receptor thereof in the stomach. Accordingly, the inhibitor of the present invention can specifically eradicate Hp from the stomach without the occurrence of drug-resistance, can effectively suppress gastritis, gastric ulcers etc. This effects of elimination of Hp can e enhanced with the combination of the inhibitor of gastric acid secretion.

## Claims

1. An inhibitor of Helicobacter pylori colonization, comprising as an active ingredient a polysaccharide having sulfate groups, the polysaccharide being other than polysaccharides comprising at least 50 mole % of fucose and having sulfate groups.

2. An inhibitor composition of Helicobacter pylori colonization, comprising as active ingredients a polysaccharide having sulfate groups and an inhibitor of gastric acid secretion.

3. An inhibitor according to claim 1 or claim 2, wherein the polysaccharide having sulfate groups is dextran sulfate, κ-carrageenan, λ-carrageenan, heparin or sulfated curdlan.

4. A pharmaceutical composition useful in the prevention or treatment of gastritis, gastric ulcers, and duodenal ulcers caused by Helicobacter pylori, comprising as an active ingredient a polysaccharide having sulfate groups, the polysaccharide being other than polysaccharides comprising at least 50 mole % of fucose and having sulfate groups.

5. A pharmaceutical composition useful in the prevention or treatment of gastritis, gastric ulcers, and duodenal ulcers caused by Helicobacter pylori, comprising as active ingredients a polysaccharide having sulfate groups and an inhibitor of gastric acid secretion.

6. A pharmaceutical composition according to claim 4 or claim 5, wherein the polysaccharide having sulfate groups is dextran sulfate, κ-carrageenan, λ-carrageenan, heparin or sulfated curdlan.

7. A food useful in the prevention or treatment of gastritis, gastric ulcers, and duodenal ulcers, containing the inhibitor according to Claim 1.

8. A food according to claim 7, wherein the polysaccharide having sulfate groups is dextran sulfate, κ-carrageenan, λ-carrageenan, heparin or sulfated curdlan.
